# EUROPÄISCHE PATENTSCHRIFT

(11) **EP 0 558 727 B1**
(45) Veröffentlichungstag und Bekanntmachung des Hinweises auf die Patenterteilung: **02.02.2000**
(21) Anmeldenummer: 92919963.6
(22) Anmeldetag: 09.09.1992
(51) Int. Cl.: A61L 24/00, A61K 38/00, A61K 38/27

(54) **KNOCHENWACHSTUMSFÖRDERNDE ZUSAMMENSETZUNG**
BONE-GROWTH-STIMULATING COMPOSITION
COMPOSITION STIMULANT LA CROISSANCE OSSEUSE

(30) Priorität: 13.09.1991 DE 4130545
(43) Veröffentlichungstag der Anmeldung: 08.09.1993
(73) Patentinhaber: Baylink, David Jeston, Prof. Dr., Loma Linda CA 92357 (US); Ewers, Rolf, Prof. Dr. Dr., A-1160 Wien (AT); Kirsch, Axel, Dr., 70794 Filderstadt (DE)
(72) Erfinder: Baylink, David Jeston, Prof. Dr., Loma Linda CA 92357 (US); Ewers, Rolf, Prof. Dr. Dr., A-1160 Wien (AT); Kirsch, Axel, Dr., 70794 Filderstadt (DE)
(74) Vertreter: Goddar, Heinz J., Dr.
(86) Internationale Anmeldenummer: DE9200778
(87) Internationale Veröffentlichungsnummer: WO9305808

(56) Entgegenhaltungen:
- EP-A- 0 322 249
- WO-A-90/04974
- US-A- 5 118 667

## Beschreibung

Die Erfindung betrifft die Verwendung einer knochenwachstumsfördernden Zusammensetzung.

Die Knochenbildung bei Wirbeltieren ist ein dynamischer Prozeß, der die fortlaufende Knochenproduktion und Knochenresorption umfaßt. Knochenheilung erfordert die Vermehrung neuer Osteoblasten, die sich ihrerseits differenzieren und Knochenmatrix synthetisieren. Bei Knochendefekten verwendet man normalerweise autologes Knochen-Pfropfmaterial. Der Nachteil dieses Ansatzes liegt darin, daß autologes Knochenmaterial die Heilung nicht so schnell beschleunigen kann, wie es erwünscht wäre, und daß in einigen Fällen autologes Knochenmaterial nicht verfügbar ist.

Unter den pharmazeutischen Zusammensetzungen, die verwendet worden sind, um Knochenerkrankungen zu behandeln, findet sich Fluorid, von dem gezeigt worden ist, daß es die Knochenbildung in vivo durch einen direkten mitogenen Effekt auf Knochenzellen stimuliert. Die Dichte von Knochenmineral des axialen Skeletts von Patienten, die mit Fluorid behandelt worden sind, steigt an, ebenso wie der Serumspiegel an alkalischer Phosphatase, einem Index für Knochenbildung im Skelett. Signifikante Anstiege in der Dichte der Wirbelsäulenknochen können nach etwa 12-18 Monaten Fluorid-Therapie beobachtet werden. Die Fluorid-Behandlung ist jedoch nicht bei allen Patienten gleich wirksam, da einige Patienten nur sehr schwach auf Fluorid reagieren und andere überhaupt nicht.

Zusätzlich zur Fluorid-Behandlung gibt es eine Anzahl anderer Techniken, die für den Zuwachs von Knochenmasse vorgeschlagen worden sind, einschließlich der Verwendung von 1,25-Dihydroxy-Vitamin E und anderen wachstumsfördernden Substanzen, aber diese Behandlungen haben teilweise nur sehr unbestimmte Erfolge gezeigt.

Die europäische Patentanmeldung 0 289 314 offenbart die Verwendung von IGF-II bei der Behandlung von Knochenstörungen. Außerdem ist offenbart, daß die Kombination von IGF-II und Fluorid-Ionen eine unerwartet verstärkte Wirkung auf die Vermehrung der Knochenzellen bewirkt, wobei das Fluorid-Ion die die Osteoblasten stimulierende Wirkung von IFG-II verstärkt.

WO90/04974 offenbart ein Verfahren zur Behandlung von Zahnwurzelhautentzündung, bei dem TGF-β und desweiteren ein chemotherapeutisches Agens, wie z. B. Zinnfluorid, verwendet wird.

EP 0 514 520 offenbart ein Verfahren zur Förderung der Knochenneubildung durch Verabreichung einer Kombination eines Knochenwachstumsfaktors und eines Knochenresorptionsinhibitors. Als Knochenwachstumsfaktoren wird, unter anderem, TGF-β und als Knochenresorptionsinhibitor Natriumfluorid offenbart.

Der vorliegenden Erfindung liegt die Aufgabe zugrunde, für eine Zusammensetzung mit einem Gehalt an wenigstens einer Substanz aus der Gruppe, die aus den Wachstumsfaktoren FGF, TGF-β, IGF-II und PDGF besteht, sowie wenigstens einem als Phosphotyrosylproteinphosphataseinhibitor wirkenden Salz aus der Gruppe, die aus physiologisch verträglichen Vanadaten, Molybdaten und Fluoriden besteht, ausgenommen die Kombination aus IGF-II und Fluorid oder Orthovanadat sowie TGF-β und Fluorid, eine Verwendung vorzusehen.

Erfindungsgemäß wird die Aufgabe dadurch gelöst, daß die vorstehend genannte Zusammensetzung zur Herstellung eines Arzneimittels zur Behandlung von Knochenerkrankungen verwendet wird.

Weitere bevorzugte Ausführungsformen ergeben sich aus den Unteransprüchen.

Zu unserer eigenen Überraschung konnte gezeigt werden, daß nicht nur die Kombination aus IGF-II und Fluorid bei der Steigerung der Vermehrung von Knochenzellen wirksam ist, sondern daß jede der vorgenannten Kombinationen aus wenigstens einem der genannten Wachstumsfaktoren (oder einem biologisch aktiven Mutanten oder Fragment derselben) und wenigstens einem der genannten Salze in dieser Hinsicht in wenigstens vergleichbarem Maße wirksam ist.

In einem ersten Versuch, diese Befunde zu erklären, vermuten wir, daß die Verstärkungswirkung von Fluorid-, Vanadat-, oder Molybdatverbindungen auf ihrer hemmenden Wirkung auf Phosphotyrosylproteinphosphatase beruht. Dieses Enzym bewirkt die Dephosphorylierung von Phosphoproteinen, induziert von mitogenen Signalen, wodurch diese mitogenen Signale inaktiviert werden. Durch Hemmen dieser Dephosphorylierung verstärkt man dann das mitogene Signal, das vom Wachstumstaktor erzeugt wird.

In einer bevorzugten Ausführungsform der Erfindung enthält die verwendete Zusammensetzung in Kombination wenigstens zwei Wachstumsfaktoren, am bevorzugtesten drei, von denen einer oder mehrere fakultativ durch eine biologisch aktive Mutante oder ein entsprechendes Fragment ersetzt sind. Für diese Kombinationen konnte gezeigt werden, daß die Kombination von zwei oder mehr Wachstumsfaktoren (oder entsprechender Mutanten oder Fragmente derselben) nicht nur eine additive Wirkung auf die Vermehrung und Differenzierung von Knochenzellen hat, sondern einen überraschend merkbaren Synergismus schafft.

Der Wachstumsfaktor zur Verwendung in einer Zusammensetzung nach der vorliegenden Erfindung kann mit einer Anzahl verschiedener Techniken hergestellt werden.

Erstens schlägt die vorliegenden Erfindung vor, daß wenigstens einer der Wachstumsfaktoren aus natürlichem, vorzugsweise menschlichem Knochenmaterial gewonnen ist, wie z.B. für IGF-II von Mojan et al., Biochem. Biophys. Akta 884, 234 (1986) beschrieben. Alternativ dazu können die Wachstumsfaktoren auch aus Knochenzellen isoliert werden, die in Kultur gezüchtet sind.

Der zweite Weg für die Gewinnung der Wachstumsfaktoren ist die Isolierung aus Serum, einschließlich Human-, Rinder-, Scharfs-, Schweine- oder Pferdeserum, unter Verwendung herkömmlicher Techniken.

Die dritte (und möglicherweise letztendlich die bevorzugte) Herstellungmethode umfaßt jedoch rekombinante DNA-Techniken, d.h. die Gewinnung der Wachstumsfaktoren oder biologisch aktiven Mutanten oder Fragmente derselben als rekombinante Verbindung aus einem gentechnologischen Verfahren. Einige dieser Techniken sind zum Beispiel für IGF-II in obengenannter europäischer Patentanmeldung 0 289 314 beschrieben.

Schließlich kann die erfindungsgemäß verwendete Zusammensetzung noch durch einen Gehalt an einem mitogenen Knochenextrakt gekennzeichnet sein. Die Herstellung eines solchen wirksamen Knochenextrakts, der ein oder mehrere der genannten Wachstumsfaktoren enthält, ist unter Verwendung üblicher Techniken möglich.

Bei der erfindungsgemäßen Verwendung der knochenwachstumsfördernden Zusammensetzung wird die Wirkung der Wachstumsfaktoren gefördert, was zu einer Vermehrung und Differenzierung der Knochenzellen fuhrt. Diese neuen Knochenzellen verwirklichen dann das Ziel, entweder Knochendefekte schnell aufzufüllen oder das Heilen von zum Beispiel unregelmäßigen Brüchen zu fördern. Die erfindungsgemäße Verwendung erstreckt sich dabei auch auf die Verwendung bei der Herstellung von Arzneimittel zur Behandlung von anderen Knochenerkrankungen, wie Osteoporose.

Die Erfindung wird nun anhand der folgenden Beispiele noch weiter veranschaulicht.

### BEISPIEL 1

### Wirkung von TGF-β (oder IGF-II) und Vanadat auch die Vermehrung und Differenzierung von menschlichen Knochenzellen

Menschliche Osteosarcomzellen, SaOs, wurden als Schicht auf DMEM aufgebracht, das 1% Kalbsserum enthält. Am folgenden Tag wurde das Medium gegen serumfreies DMEM ausgetauscht. Am nächsten Tag wurde die Faktoren zugesetzt (d.h. TGF-β (oder IGF-II) und Vanadat). Nach 18 Stunden wurde eine Zellvermehrung durch Einbringen von ³H-Thymidin festgestellt. Parallele andere Platten wurden einmal mit PBS gespült und die Zellschicht mit 0,01% Triton X-100 extrahiert. Die Extrakte wurden auf Protein und Aktivität von alkalischer Phosphatase hin untersucht. Die Ergebnisse zeigen klar, daß TGF-β (und IGF-II) die Wirkung von Vanadat auf die Vermehrung von menschlichen Knochenzellen steigert.

### BEISPIEL 2

### Wirkung von FGF und Molybdat auf die Vermehrung und Differenzierung von menschlichen Knochenzellen

Die Vorgehensweise dieses Beispiels ist vergleichbar mit Beispiel 1, mit der Ausnahme, daß die entsprechenden anderen Faktoren zugesetzt werden. Wieder werden die Extrakte auf Protein und Aktivität von alkalischer Phosphatase untersucht. Auch bei FGF zeigt sich, daß die Wirkung von Molybdat auf die Vermehrung von menschlichen Knochenzellen gesteigert wird.

### BEISPIEL 3

### Wirkung von PDGF und Fluorid auf die Vermehrung und Differen

zierung von menschlichen Knochenzellen Die Vorgehensweise dieses Beispiels ist vergleichbar mit Beispiel 1, mit der Ausnahme, daß die entsprechenden anderen Faktoren zugesetzt werden. Auch hier werden die Extrakte wieder auf Protein und Aktivität von alkalischer Phosphatase hin untersucht. Auch PDGF steigert die Wirkung von Fluorid auf die Vermehrung von menschlichen Knochenzellen.

## Patentansprüche

1. Verwendung einer knochenwachstumsfördernden Zusammensetzung mit einem Gehalt an wenigstens einer Substanz aus der Gruppe, die aus den Wachstumsfaktoren FGF, TGF-β, IGF-II und PDGF besteht, sowie wenigstens einem als Phosphotyrosylproteinphosphataseinhibitor wirkenden Salz aus der Gruppe, die aus physiologisch verträglichen Vanadaten, Molybdaten und Fluoriden besteht, ausgenommen die Kombination aus IGF-II und Fluorid oder Orthovanadat sowie TGF-β und Fluorid, zur Herstellung eines Arzneimittels für die Behandlung von Knochenerkrankungen.

2. Verwendung nach Anspruch 1, dadurch gekennzeichnet, daß die knochenwachstumsfördernde Zusammensetzung in Kombination wenigstens zwei Wachstumsfaktoren enthält.

3. Verwendung nach Anspruch 2, dadurch gekennzeichnet, daß die knochenwachstumsfördernde Zusammensetzung in Kombination drei Wachstumsfaktoren enthält.

4. Verwendung nach einem der Ansprüche 1 bis 3, dadurch gekennzeichnet, daß das mit dem Vanadat-, Molybdat- oder Fluorid-Ion verknüpfte Kation ausgewählt ist aus der Gruppe, die aus Natrium, Kalium, Calcium, Magnesium und Ammonium besteht.

5. Verwendung nach einem der vorangehenden Ansprüche, dadurch gekennzeichnet, daß wenigstens einer der Wachstumsfaktoren aus natürlichem Knochenmaterial gewonnen ist.

6. Verwendung nach Anspruch 5, dadurch gekennzeichnet, daß wenigstens einer der Wachstumsfaktoren aus menschlichem Knochenmaterial gewonnen ist.

7. Verwendung nach einem der vorangehenden Ansprüche, dadurch gekennzeichnet, daß wenigstens einer der Wachstumsfaktoren aus Serum, einschließlich Human-, Rinder-, Schafs-, Schweine- oder Pferdeserum, gewonnen ist.

8. Verwendung nach einem der vorangehenden Ansprüche, dadurch gekennzeichnet, daß wenigstens einer der Wachstumsfaktoren als rekombinante Verbindung aus einem gentechnologischen Verfahren gewonnen ist.

9. Verwendung nach einem der vorangehenden Ansprüche, gekennzeichnet durch einen Gehalt an einem mitogenen Knochenextrakt.

## Claims

1. Use of a bone growth promoting composition containing at least one substance from the group consisting of the growth factors FGF, TGF-β, IGF-II and PDGF, and at least one salt acting as a phosphotyrosyl protein phosphatase inhibitors, said salt being selected from the group consisting of physiologically compatible vanadates, molybdates and fluorides, excluding the combination of IGF-II and fluoride or orthovanadate and TGF-β and fluoride, for the preparation of a medicament for the treatment of bone diseases.

2. Use according to claim 1, characterised in that the bone growth promoting composition contains at least, two growth factors in combination.

3. Use according to claim 2, characterised in that the bone growth promoting composition contains three growth factors in combination.

4. Use according to any one of claims 1 to 3, characterised in that the cation linked with the vanadate, molybdate or fluoride ion is selected from the group consisting of sodium, potassium, calcium, magnesium and ammonium.

5. Use according to any one of the preceding claims, characterised in that at least one of the growth factors is obtained from natural bone material.

6. Use according to claim 5, characterised in that at least one of the growth factors is obtained from human bone material.

7. Use according to any one of the preceding claims, characterised in that at least one of the growth factors is obtained from serum, including human, bovine, sheep, pig or horse serum.

8. Use according to any one of the preceding claims, characterised in that at least one of the growth factors is obtained as a recombinant compound from a genetic engineering process.

9. Use according to any one of the preceding claims, characterized by a content of a mitogenic bone extract.

## Revendications

1. Utilisation d'un composé favorisant la croissance, avec une proportion d'au moins une substance provenant du groupe constitué des facteurs de croissance FGF, TGF-β, IGF-II et PDGF, ainsi qu'avec au moins un sel à effet inhibiteur de phosphatase à protéine phosphotyrosyle provenant du groupe constitué des vanadates, molybdates et fluorures physiologiquement compatibles, à l'exception de la combinaison constituée d'IGF-II et de fluorure ou d'orthovanadate ainsi que de TGF-β et de fluorure, en vue de fabriquer un médicament pour traiter les maladies osseuses.

2. Utilisation selon la revendication 1, caractérisée en ce que le composé favorisant la croissance contient en combinaison au moins deux facteurs de croissance.

3. Utilisation selon la revendication 2, caractérisée en ce que le composé favorisant la croissance contient en combinaison trois facteurs de croissance.

4. Utilisation selon l'une des revendications 1 à 3, caractérisée en ce que le cation combiné avec l'ion vanadate, molybdate ou fluorure est choisi dans le groupe constitué du sodium, du potassium, du calcium, du magnésium et de l'ammonium.

5. Utilisation selon une des revendications précédentes, caractérisée en ce qu'au moins un des facteurs de croissance est obtenu à partir de matière osseuse naturelle.

6. Utilisation selon la revendication 5, caractérisée en ce qu'au moins un des facteurs de croissance est obtenu à partir de matière osseuse humaine.

7. Utilisation selon une des revendications précédentes, caractérisée en ce qu'au moins un des facteurs de croissance est obtenu à partir de sérum, notamment de sérum humain, bovin, ovin, porcin ou équin.

8. Utilisation selon une des revendications précédentes, caractérisée en ce qu'au moins un des facteurs de croissance est obtenu, en tant que liaison recombinante, par un procédé génétique.

9. Utilisation selon une des revendications précédentes, caractérisé par une proportion d'extrait osseux mitogène.
